# EUROPEAN PATENT APPLICATION

(11) **EP 4 327 824 A1**
(43) Date of publication of application: **28.02.2024**
(21) Application number: 22472005.2
(22) Date of filing: 22.08.2022
(51) Int. Cl.: A61K 41/00, A61K 9/127, A61K 31/4015, A61K 31/409, A61K 47/00, A61P 17/10, A61Q 19/00

(54) **A TOPICAL COMPOSITION COMPRISING CHLORINE E6 AND ZINC L-PYROGLUTAMATE**

(71) Applicant: ZXA Group Ltd., 1000 Sofia (BG)
(72) Inventor: Galstyan Sargsyan, Ruzana, 03820 Cocentaina, Alicante (ES)
(74) Representative: ZBM Patents - Zea, Barlocci & Markvardsen

(57) **Abstract**

The present invention relates to a topical composition comprising a) chlorine e6, or a topically acceptable salt thereof; or a topically acceptable liposome containing it; b) zinc salt of *L*-pyroglutamine acid; and c) optionally, one or more topically acceptable excipients or carrier. It also relates to processes for its preparation, and its use in therapy and cosmetics, and also as photosensitizing agent; particularly in therapeutic and cosmetic photodynamic therapy.

## Description

The present invention relates to a composition comprising chlorine e6. Particularly, to a topical composition comprising chlorine e6 and a zinc salt of L-pyroglutamine acid; to processes for their preparation as well as to its use in therapy and cosmetics. It also relates to the use of the composition as sensitizing agent in photodynamic therapy.

### Background Art

Chlorine e6 is known in the art for its beneficial effect as skin care agent, and also in the treatment of disease or conditions of skin. The chlorin e6 is the non-proprietary name of the compound (17S,18S)-18-(2-carboxyethyl)-20-(carboxymethyl)-12-ethenyl-7-ethyl-3,8,13,17-tetramethyl-17,18,22,23-tetrahydroporphyrin-2-carboxylic acid having the CAS number 19660-77-6. Chlorin e6 corresponds to the compound of formula (I):

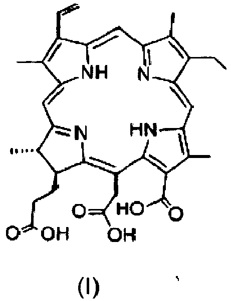

However, it is known in the art that chlorine e6 has some disadvantages related to an inefficient delivery into the skin making it difficult to use effectively topically effective. Thus, in order to solve this problem, compositions comprising liposomes of chlorine e6 have been disclosed in the state of the art. In fact, in the US patent application number US2016213781, a conjugate of chlorine e6 with lysophosphatidylcholine (LPPC-Ce6) has been disclosed. This conjugate allows ameliorate the transfer of the chlorin e6 into the skin. However, an erratic effect against the treatment of acne is still observed when the liposome of chlorine e6 is used mainly due to the decreased hydrophilicity of the liposomes.

Furthermore, it is also observed that at low amount of the liposome, a weak therapeutic effect is observed, but if the amount of the liposomes is increased in the topical composition, the cost of the treatment is drastically and undesirable increased without showing a proportional increase in the beneficial effect of the chlorine e6 against skin disease or disorders such as acne. Therefore, the use of the liposome of chlorine e6 does not effectively solve the skin penetration capability of chlorine e6.

Therefore, from what is known in the art, there is still the need of providing a more effective topical composition of chlorine e6 as skin care agent or for the treatment of skin diseases or disorders.

### Summary of Invention

Inventors have found that the combination of chlorine e6 and zinc salt of L-pyroglutamine acid promote an enhanced beneficial effect in the skin, bigger that those observed separately. In fact, the composition of the present invention has an unexpected enhanced beneficial effect in the general appearance of the skin, particularly in those skins suffering from a disease or condition that involves a disturbance of the sebaceous gland secretion.

As it is demonstrated in the experimental section, the composition of the present invention that comprise the combination of chlorine e6 and zinc salt of L-pyroglutamine acid allows a significant reduction of the inflammatory eruptions, and also a stronger improvement of the resorption of closed comedones was observed. Further, a significant improvement in the microrelief, skin texture and increased luminosity in the treated area with the composition of the invention is also seen in comparison with the untreated area.

On the other hand, a considerable amelioration in complexion and smoothing of scar tissue in the treated are with the composition of the present invention was also noted. In fact, the general rash of the skin was completely stopped.

Finally, according to the patient's evaluation, the skin had become more elastic and a generalized improvement of the skin was observed after a few sessions having a good cosmeticity, and a very satisfactory acceptability for the users. In fact, after its application, the composition is uniformly distributed and without providing any oily or sticky feeling, leaving (after the phototherapy) a dry skin with a comfortable feeling and clean appearance.

Therefore, the inventors have provided a topical composition comprising the combination of chlorine e6 and the zinc salt of *L*-pyroglutamine acid for use in therapy or cosmetic. In particular, as photosensitising agent in therapeutic or cosmetic photodynamic therapy.

Thus, the first aspect of the invention relates to a topical composition comprising: A topical composition comprising: a) an effective amount of 18-carboxy-20- (carboxymethyl) -8-ethenyl-13-ethyl-2,3-dihydro-3,7,12,17-tetramethyl 21H, 23H porphin-2-propionic acid of formula (I): 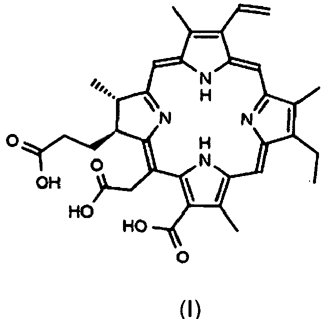 or a topically acceptable salt thereof; or a topically acceptable liposome containing it; b) an effective amount of zinc salt of *L*-pyroglutamine acid of formula (V) 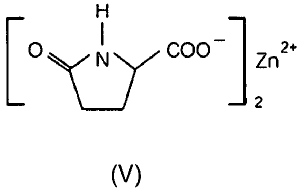 ; and
c) optionally, one or more topically acceptable excipients or carriers.

The second aspect of the present invention relates to a composition of the first aspect of the invention, which is a pharmaceutical composition comprising: a) a therapeutically effective amount of chlorine e6, or a topically acceptable salt thereof; or a topically acceptable liposome containing it; b) a therapeutically effective amount of zinc salt of L-pyroglutamine acid; and c) optionally, one or more topically pharmaceutically acceptable excipients or carriers; for use in therapy.

The third aspect of the invention relates to the use of a composition of the first aspect of the invention which is a cosmetic composition comprising: a) a cosmetically effective amount of chlorine e6, or a topically acceptable salt thereof; or a topically acceptable liposome containing it; b) a cosmetically effective amount of zinc salt of *L*-pyroglutamine acid; and c) optionally, one or more topically cosmetically acceptable excipients or carriers; in cosmetic. And, particularly as a skin care agent.

The fourth aspect of the present invention relates to the use of a composition of the first aspect of the invention as photosensitizing agent; particularly in a photodynamic therapy.

And the fifth aspect of the present invention relates to a kit comprising the composition of the first aspect of the invention; optionally means to apply the composition to the area to be treated; and optionally, a light emission source.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper endpoints of the range. Ranges given, such as weight, temperatures, times, weights, and the like, should be considered approximate, unless specifically stated.

The terms "percentage (%) by weight", "weight/weight %" and "w/w%" have the same meaning and are used interchangeable. They refer to the percentage of each ingredient of the composition in relation to the total weight of the composition.

As it is mentioned above, the topical composition of the first aspect of the invention comprises: a) an effective amount of 18-carboxy-20- (carboxymethyl) -8-ethenyl-13-ethyl-2,3-dihydro-3,7,12,17-tetramethyl 21H, 23H porphin-2-propionic acid of formula (I); or a topically acceptable salt thereof; or a topically acceptable liposome containing it; b) an effective amount of zinc salt of *L*-pyroglutamine acid of formula (V); and c) optionally, one or more topically acceptable excipients or carriers.

The term "effective amount" refers to the amount of the active ingredient which provide a therapeutic or cosmetic effect after its application. For the purpose of the present invention, the expression "effective amount of chlorine e6" encompasses the appropriate amount of salt of chlorine e6 or liposome containing the chlorine e6 that renders the therapeutic or cosmetic effective amount of chlorine e6 in free form.
As it is mentioned above, the topical composition comprises a) an effective amount of chlorine e6 of formula (I) or a topically acceptable salt thereof; or a topically acceptable liposome containing it. For the purpose of the present invention, the term "chlorin e6" is the non-proprietary name of the compound (17S,18S)-18-(2-carboxyethyl)-20-(carboxymethyl)-12-ethenyl-7-ethyl-3,8,13,17-tetramethyl-17,18,22,23-tetrahydroporphyrin-2-carboxylic acid having the CAS number 19660-77-6 and corresponding to the compound of formula (I) as disclosed above. In an embodiment, the topical composition comprises a) from 0.01% to 1% by weight of chlorine e6 of formula (I) or such amount of chlorine salt or liposome containing it equivalent to an amount from 0.01% to 1% by weight. In an embodiment, the topical composition comprises a) from 0.03% to 0.6% by weight of chlorine e6 of formula (I) or such amount of chlorine salt or liposome containing it equivalent to an amount from 0.01% to 1% by weight.

In an embodiment, the topical composition comprises a) an effective amount of a topically acceptable salt of chlorine e6. For the purpose of the present invention the term "acceptable salt of chlorine e6" refers to its pharmaceutically or cosmetically acceptable salt of chlorine e6. There is no limitation on the type of chlorine e6 salt that can be used, as long as they are pharmaceutically or cosmetically acceptable when used for therapeutic purposes or cosmetic purposes, respectively. Chlorine e6 and a salt thereof may differ in some physical properties but they are equivalent for the purposes of the present invention and the skin-friendly properties of chlorine e6 are extensible to a pharmaceutically or cosmetically acceptable salt. The term "pharmaceutically acceptable salt" refers to the salt appropriate for use in pharmaceutical technology for the preparation of compositions for medical use and, the term "cosmetically acceptable salt" used interchangeably in this document refers to the salt appropriate for use in human skin contact without toxicity, incompatibility, instability, inappropriate allergic response, among others. In particular, the term "pharmaceutical or cosmetically acceptable salt" covers commonly used salts. Non-limiting examples of pharmaceutical or cosmetically acceptable salts of chlorine e6 include alkaline metal salts such as trisodium salt; alkaline earth metal salts; and trimeglumine salt.

In an embodiment, the topical composition comprises a) an effective amount of a topically acceptable salt of chlorine e6 of formula (I), which is the trisodium salt of formula (II)

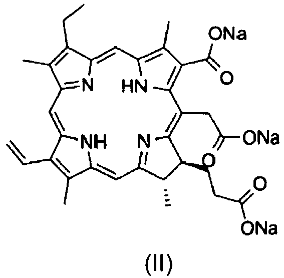

In an embodiment, the topical composition comprises a) an effective amount of a topically acceptable salt of chlorine e6 of formula (I), which is the trimeglumine salt of formula (III)

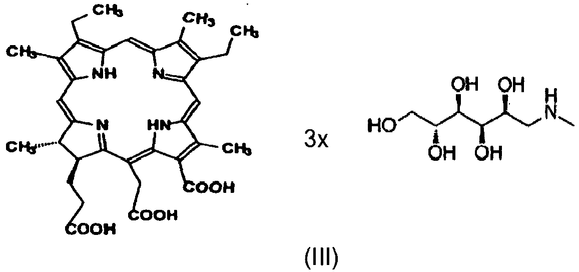

For the purpose of the invention, the term meglumine is the non-proprietary name of the compound (2*R*,3*R*,4*R*,5*S*)-6-(methylamino) hexane-1,2,3,4,5-pentol having the following formula:

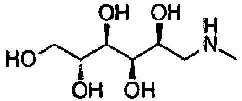

The preparation of acceptable salts of chlorine e6 can be carried out by methods known in the technique. The appropriate reagents and their amounts, as well as the reaction condition can readily be determined by those skilled in the art according to the type of salt being prepared.

In an embodiment, the topical composition comprises a) an effective amount of a liposome containing of chlorine e6 of formula (I). In an embodiment, the topical composition comprises a) an effective amount of a liposome containing of chlorine e6.

The preparation of acceptable liposomes of chlorine e6 can be carried out by methods known in the technique. The appropriate reagents and their amounts, as well as the reaction condition can readily be determined by those skilled in the art according to the type of salt being prepared.

As it is mentioned above, the topical composition comprises b) an effective amount of zinc salt of *L*-pyroglutamine acid of formula (V) as defined above. The term "zinc salt of L-pyroglutamine acid", "zinc salt of pyrrolidone carboxylic acid" and the abbreviature "Zinc-PCA" have the same meaning and they are use interchangeable. They refer to the compound having the CAS number 15454-75-8 and the following chemical structure:

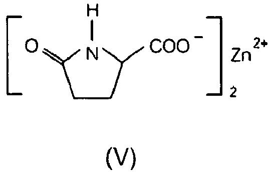

In an embodiment, the topical composition comprises b) from 0.01% to 2 % by weight of Zinc-PCA. In an embodiment, the topical composition comprises b) from 0.05% to 0.3% by weight of Zinc-PCA.

In an embodiment, the topical composition comprises:
a) From 0.01% to 1% by weight of chlorine e6, or a topically acceptable salt thereof, or a topically acceptable liposome containing it; and
b) From 0.01 % to 2 % by weight of the zinc-PCA.

In an embodiment, the topical composition comprises:
a) From 0.03% to 0.6% by weight of chlorine e6, or a topically acceptable salt thereof, or a topically acceptable liposome containing it; and
b) From 0.05% to 0.3% by weight of the zinc-PCA.

As it is mentioned above, the topical composition optionally comprises c) one or more topically acceptable excipients or carrier. In an embodiment, the topical composition comprises c) one or more topically acceptable excipients or carriers. For the purpose of the present invention, the terms "topically acceptable excipient or carrier" refers to that excipients or carriers suitable for use in contact with human skin without undue toxicity, incompatibility, instability, allergic response, among others. The appropriate excipients and/or carriers, and their amounts, can readily be determined by those skilled in the art according to the type of formulation being prepared.

In an embodiment, the topical composition comprises:
a) From 0.01% to 1% by weight of chlorine e6, or a topically acceptable salt thereof, or a topically acceptable liposome containing it;
b) From 0.01 % to 2 % by weight of the zinc-PCA; and
c) optionally, one or more topically acceptable excipients or carriers.

In an embodiment, the topical composition comprises:
a) From 0.01% to 1% by weight of chlorine e6, or a topically acceptable salt thereof, or a topically acceptable liposome containing it;
b) From 0.01 % to 2 % by weight of the zinc-PCA; and
c) one or more topically acceptable excipients or carriers.

In an embodiment, the topical composition comprises:
a) From 0.03% to 0.6% by weight of chlorine e6, or a topically acceptable salt thereof, or a topically acceptable liposome containing it;
b) From 0.05% to 0.3% by weight of the zinc-PCA; and
c) optionally, one or more topically acceptable excipients or carriers.

In an embodiment, the topical composition comprises:
a) From 0.03% to 0.6% by weight of chlorine e6, or a topically acceptable salt thereof, or a topically acceptable liposome containing it;
b) From 0.05% to 0.3% by weight of the zinc-PCA; and
c) one or more topically acceptable excipients or carriers.

In an embodiment, the topical composition comprises one or more topically acceptable excipients or carriers selected from the group consisting of solvent, co-solvent, emollient, humectant, emulsifying agent, stabilizer, preservative, parfum, antioxidant, rheology modifier agent, pH regulator agent and gelling agent.

The term "emollient" agent refers to a material that softens and soothes the skin in order to correct dryness and scaling of the skin, lubricating the skin surface, encouraging skin water retention, and altering product textures. Examples of appropriate topical emollient agents include, but are not limited to, octyl hydroxystearate, lanolin, caprylic/capric triglyceride, cetyl palmitate, octyldodecanol, cetyl alcohol, isopropyl isostearate, glyceryl dilaurate, isopropyl myristate, palm alcohol, dimethicone, squalane, plukenetia volubilis seed oil, butyrospermum parkii butter, sucrose cocoate, or their mixtures. Preferably the emollient is selected from the group consisting of dimethicone, squalane, plukenetia volubilis seed oil, butyrospermum parkii butter, caprylic/capric triglyceride, octyldodecanol, or their mixtures

The term "humectant" agent refers to a hygroscopic material which attracts water molecules from the surrounding environment though either absorption or adsorption, preventing the skin from losing moisture. Examples of appropriate topical humectants include, but are not limited to, glycerin, diglycerin, ethylhexylglycerin, glucose, honey, lactic acid, polyethylene glycol, propylene glycol, sorbitol, sucrose, or threalose. Preferably, the humectant is selected group consisting of glycerin, diglycerin, ethylhexylglycerin, and their mixtures.

The term "emulsifying agent" or "emulsifier" which is herein used interchangeably refers to a material that reduces surface tension, promoting the formation of intimate mixtures of non-miscible liquids by altering the interfacial tension. Emulsifier stabilizes an emulsion by increasing its kinetic stability. Examples of appropriate emulsifier include, but are not limited to, glyceryl trioleate, glyceryl oleate, acetylated sucrose distearate, sorbitan trioleate, polyoxyethylene monostearate, glycerol monooleate, sucrose distearate, polyethylene glycol monostearate, octyl phenoxypoly (ethyleneoxy) ethanol, deacylerin penta-isostearate, sorbitan sesquioleate, hydroxylated lanolin, lecithin, lanolin, triglyceryl diisostearate, polyoxyethylene oleyl ether, calcium stearoyl-2-lactylate, sodium lauroyl lactylate, sodium stearoyl lactylate, cetearyl glucoside, methyl glucoside sesquistearate, sorbitan monopalmitate, methoxy polyethylene glycol-22/dodecyl glycol copolymer, polyethylene glycol-45/dodecyl glycol copolymer, polyethylene glycol 400 distearate and glyceryl stearate, candelilla/jojoba/rice bran polyglyceryl-3 esters, cetyl phosphate, potassium cetyl phosphate, or their mixtures. Preferably, the emulsifier is selected group consisting of glyceryl oleate, lecithin, sodium lauroyl lactylate, sodium stearoyl lactylate, glyceryl stearate, candelilla/jojoba/rice bran polyglyceryl-3 esters, and their mixtures.

The term "stabilizer" refers to a material which lowers the surface tension of a liquid and the interfacial tension between two liquids, allowing their easier spreading. Surfactants have a hydrophilic head that is attracted to water molecules and a hydrophobic tail that repels water and simultaneously attaches itself to oil and grease in dirt. These opposing forces loosen the dirt and suspend it in the water, having the ability to remove it from surfaces such as the human skin, textiles, and other solids, when surfactants are dissolved in water. Examples of appropriate surfactant agents include, but are not limited to, non-ionic, ionic (either anionic or cationic) or zwitterionic (or amphoteric wherein the head of the surfactant contains two oppositely charged groups) surfactants. Examples of anionic surfactants include, but are not limited to, those based on sulfate, sulfonate or carboxylate anions such as perfluorooctanoate (PFOA or PFO), alkyl benzene sulfonate, soaps, fatty acid salts, or alkyl sulfate salts such as perfluorooctanesulfonate (PFOS), sodium dodecyl sulfate (SDS), ammonium lauryl sulfate, or sodium lauryl ether sulfate (SLES). Examples of cationic surfactants include, but are not limited to, those based on quaternary ammonium cations such as or alkyltrimethylammonium including cetyl trimethylammonium bromide (CTAB) a.k.a., or hexadecyl trimethyl ammonium bromide, cetylpyridinium chloride (CPC), polyethoxylated tallow amine (POEA), benzalkonium chloride (BAC), or benzethonium chloride (BZT). Examples of zwitterionic surfactants include, but are not limited to dodecyl betaine, cocamidopropyl betaine, or coco ampho glycinate. Examples of non-ionic surfactants include, but are not limited to, alkyl poly(ethylene oxide), alkylphenol poly(ethylene oxide), copolymers of poly(ethylene oxide), polypropylene oxide) (commercially called Poloxamers or Poloxamines), alkyl polyglucosides including octyl glucoside and decyl maltoside, fatty alcohols including cetyl alcohol and oleyl alcohol, cocamide MEA, cocamide DEA, or polysorbates including tween 20, tween 80, or dodecyl dimethylamine oxide. Preferably, the surfactant is foaming and skin friendly, including polysorbate 20 or 40, coco glucoside, lauryl glucoside, decyl glucoside, lauryl sulfates such as ammonium, sodium, magnesium, MEA, triethylamine (TEA), or mipa lauryl sulfate, cocamidopropyl betain, or sodium alkyl sulfosuccinates.

The term "preservative" refers to a material that prevents, reduces, or slows down microbial growth, providing that the stability of the solution is not affected. Examples of appropriate preservative agents include, but are not limited to, benzoic acid, butylparaben, ethylparaben, propylparaben, methylparaben, sorbic acid, potassium sorbate, sodium benzoate, phenoxyethanol, triclosan, or their mixtures.

The term "antioxidant" refers to a material that slows or prevents the oxidation of other molecules. Antioxidants include free radical scavengers and reducing agents. Examples of appropriate antioxidants include, but are not limited to, free radical scavengers or reducing agents such as, acetyl cysteine, ascorbic acid, ascorbyl palmitate, butylated hydroxytoluene, green tea extract, caffeic acid, cysteine, tocopherol, ubiquinone, propyl gallate, butylated hydroxytoluene (BHT), and their mixtures. Preferably, the antioxidant agent is selected group consisting of ascorbyl palmitate, and tocopherol.

The term "thickening agent" or "thickener" or "viscosity agent "or "rheology modifier agent" which is herein used interchangeably refers to a material that increases its viscosity without substantially modifying its other properties. Examples of appropriate viscosity agents include, but are not limited to, cellulose or their derivatives such as hydroxypropyl methylcellulose, polyethylene glycol, microcrystalline cellulose, cetearyl alcohol, alginates, branched polysaccharides, fumed silica, xanthan gum, carbomer, and polyacrylates. Preferably, the viscosity agent is selected group consisting of microcrystalline cellulose, cetearyl alcohol, cellulose, xanthan gum, and carbomer.

The term "pH-regulating" agent refers to acids or bases that can be used to adjust the pH of the finished product to the desired level, without affecting the stability of the solution. Examples of appropriate topical pH-regulating agents include, but are not limited to, acetic acid, lactic acid, citric acid, ethanolamine, formic acid, oxalic acid, potassium hydroxide, sodium hydroxide, triethanolamine, or their mixtures. Preferably, the pH-regulating agent is selected group consisting of triethanolamine, sodium hydroxide, lactic acid, and citric acid.

The term "gelling agent" refers to a compound that, when dissolved, suspended, or dispersed in a fluid (e.g., an aqueous fluid such as water or a buffer solution), forms a gelatinous semi-solid. Examples of gelling agents include but are not limited to hydroxyethyl cellulose, hydroxymethyl cellulose, hydroxypropyl guar, methyl cellulose, ethyl cellulose, hydroxypropyl cellulose, sodium carboxymethyl cellulose, carbomer, alginate, gelatin, and poloxamer.

The compositions mentioned above also include a vehicle (solvent and/or co-solvent). Examples of vehicles include, but are not limited to, water, propylene glycol, butylene glycol, ethanol, isopropanol, or silicones. Preferably, the vehicle is water.

Topical compositions of the present invention can be prepared according to methods well known in the state of the art. The topical compositions of the invention can be formulated in several forms that include, but are not limited to, emulsion, cream, gel, fluid, and lotion. The appropriate excipients and/or carriers, and their amounts, can readily be determined by those skilled in the art according to the type of formulation being prepared.

In an embodiment, the topical composition is a pharmaceutical composition comprising a therapeutically effective amount of the ingredients a) and b) as defined above together with one or more appropriate topical pharmaceutically acceptable excipients or carriers. The expression "therapeutically effective amount" as used herein, refers to the amount of active ingredients that, when administered, which is sufficient to prevent development of, or alleviate to some extent, one or more of the symptoms of a skin disease or condition that involves Inflammation, excessive sebum production, inflammation, and/or microbiome imbalances. The particular dose of the active ingredients administered according to this invention will of course be determined by the skilled in the art regarding the particular circumstances surrounding the case, including the particular condition being treated, and the similar considerations. And the term "pharmaceutically acceptable excipients or carriers" refers to that excipient or carrier suitable for use in the pharmaceutical technology for preparing compositions with medical use.

In an embodiment, the topical composition is a cosmetic composition comprising a cosmetically effective amount of the ingredients a) and b) as defined above together with one or more appropriate topical cosmetically acceptable excipients or carriers. The term used herein "cosmetically effective amount" refers to an amount enough to accomplish efficacies on cosmetic improvements in skin conditions described hereinabove. And the term "cosmetically acceptable" refers to that excipient or carrier suitable for use in contact with human skin without undue toxicity, incompatibility, instability, allergic response, among others for a non-medical use. The topical cosmetic composition of the present invention is designed to apply to the body to improve its appearance or to beautify, preserve, condition, cleanse, color or protect the skin, nails, or hair (cf. Academic press Dictionary of Science and Technology, 1992, pp. 531; A terminological Dictionary of the Pharmaceutical Sciences. 2007, pp.190). Therefore, the above cosmetic compositions are adjectivally used for a non-medical application.

In an embodiment, the topical composition is a medical device. For the purpose of the invention, the term "medical device" refers to any instrument, apparatus, appliance, software, implant, reagent, material, or other article intended by the manufacturer to be used, alone or in combination, for human beings for specific medical purposes as described in the current medical device legislation: Article 2, Regulation (EU) 2017/745 of The European Parliament and of The Council of 5 April 2017.

In an embodiment, the topical composition has only an aesthetic or another non-medical purpose, but similarities with medical devices in terms of functioning and risk profile, and consequently falls Annex XVI, Regulation (EU) 2017/745 of The European Parliament and of The Council of 5 April 2017.

It is also part of the invention a pharmaceutical composition of the present invention as defined above for use in therapy. In an embodiment, the pharmaceutical composition of the present invention as defined above for use in the treatment of a skin disease or disorder that involves a disturbance of the sebaceous gland secretion. This aspect could be also formulated as the use of the pharmaceutical composition of the present invention as defined above for the preparation of a medicament for the prophylaxis and/or treatment of a skin disease or condition which involves a disturbance of the sebaceous gland secretion. It also relates to a method for the prophylaxis and/or treatment of a mammal suffering or is susceptible to suffer from a skin disease or condition which involves a disturbance of the sebaceous gland secretion, the method comprises administering to said mammal a) a therapeutically effective amount of chlorine e6, or a topically acceptable salt thereof; or a topically acceptable liposome containing it; b) a therapeutically effective amount of zinc salt of *L*-pyroglutamine acid; and c) optionally, one or more topically pharmaceutically acceptable excipients or carriers. In an embodiment, the pharmaceutical composition of the present invention as defined above for use in the treatment of a skin disease or disorder that involves a disturbance of the sebaceous gland secretion selected from the group consisting of acne, rosacea, eczema, hyperpigmentation, and psoriasis.

For the purpose of the invention, the term hyperpigmentation encompasses post inflammatory trauma, melasma and age spots.

In an embodiment, the composition of the present invention is a pharmaceutical composition for use in therapy as unique therapeutic treatment or in combination with other treatments.

All embodiments as defined above for the pharmaceutically composition of the present invention in relation to the ingredients a), b) and c) also applies to its therapeutic use.

The composition of the invention can be used for reducing the normal secretion of the sebaceous glands of the skin. Thus, another aspect of the present invention is a cosmetic composition of the invention as defined above in cosmetic; particularly as skin care agent. Then, the composition comprises: a) a cosmetically effective amount of chlorine e6, or a topically acceptable salt thereof; or a topically acceptable liposome containing it;
b) a cosmetically effective amount of zinc salt of L-pyroglutamine acid; and
c) optionally, one or more topically cosmetically acceptable excipients or carriers. In an embodiment, the cosmetic use of the cosmetic composition of the present invention as skincare agent comprises ameliorating at least one of the following symptoms: roughness, flakiness, dehydration, tightness, chapping, lack of elasticity, and dull skin.

All embodiments as defined above for the cosmetic composition of the present invention in relation to the ingredients a), b) and c) also applies to its cosmetic use.

It is also a part of the invention the use of the composition of the present invention as photosensitizing agent; particularly in a photodynamic therapy. In an embodiment, the pharmaceutical composition of the present invention is useful as therapeutic photosensitizing agent; particularly in a therapeutical photodynamic therapy. In an embodiment, the cosmetic composition of the present invention is useful as cosmetic photosensitizing agent; particularly in a cosmetic photodynamic therapy.

In an embodiment, the pharmaceutical or cosmetic composition of the invention is a therapeutic or cosmetic photosensitizing agent respectively, wherein the photodynamic therapy comprises exposing the skin area to be treated to light having a wavelength from 400 to 760 nm. In an embodiment, the pharmaceutical or cosmetic composition of the invention is a therapeutic or cosmetic photosensitizing agent respectively, wherein the photodynamic therapy comprises exposing the skin area to be treated to a light having a wavelength from 410 to 670 nm.

In an embodiment, the pharmaceutical or cosmetic composition of the invention is a therapeutic or cosmetic photosensitizing agent respectively, wherein the photodynamic therapy comprises exposing the skin area to be treated to a light having a wavelength from 410 to 430 nm (also known as "blue LED light").

In an embodiment, the pharmaceutical or cosmetic composition of the invention is a therapeutic or cosmetic photosensitizing agent respectively, wherein the photodynamic therapy comprises exposing the skin area to be treated to a light having a wavelength from 580 to 600nm (also known as "yellow LED light").

In an embodiment, the pharmaceutical or cosmetic composition of the invention is a therapeutic or cosmetic photosensitizing agent respectively, wherein the photodynamic therapy comprises exposing the skin area to be treated to a light having a wavelength from 620 to 670nm (also known as "Red LED light" among other names).

In an embodiment, the pharmaceutical or cosmetic composition of the invention is a therapeutic or cosmetic photosensitizing agent respectively, wherein the photodynamic therapy comprises exposing the skin area to be treated to a light having a wavelength from 520 to 540nm (also known as "green LED light").

In an embodiment, the pharmaceutical or cosmetic composition of the invention is a therapeutic or cosmetic photosensitizing agent respectively, wherein the photodynamic therapy comprises exposing the skin area to be treated to a light having a wavelength of one or more of the previously mentioned wavelengths 410 to 430nm, 580 to 600nm and 620 to 670nm, optionally in combination with a wavelength of 520 to 540nm.

In an embodiment, the pharmaceutical or cosmetic composition of the invention is a therapeutic or cosmetic photosensitizing agent respectively, wherein the photodynamic therapy comprises exposing the skin area to be treated to a light having a wavelength of one or more of the previously mentioned wavelengths 410 to 430nm, 580 to 600nm and 620 to 670nm, in combination with a wavelength of 520 to 540nm.

In an embodiment, the pharmaceutical or cosmetic composition of the invention is a therapeutic or cosmetic photosensitizing agent respectively, wherein the photodynamic therapy comprises exposing the skin area to be treated to light having an energy from 40 to 400 joules/cm.

It is also part of the invention, a kit comprising: the composition as defined in the present invention; optionally, means to apply the composition to the area to be treated; and optionally, a light emission source. In an embodiment, the kit comprises: the composition as defined in the present invention; means to apply the composition to the area to be treated; and optionally, a light emission source. In an embodiment, the kit comprises: the composition as defined in the present invention; means to apply the composition to the area to be treated; and a light emission source. In an embodiment, the kit comprises: the composition as defined in the present invention; optionally, means to apply the composition to the area to be treated; and a light emission source. In an embodiment, the kit comprises: the composition as defined in the present invention; and a light emission source. Examples of appropriate means for the application of the composition of the invention to the area to be treated include spray, roll-on, drop tip, brush, or spatula. Examples of appropriate light emission sources for the present invention include light Sources and Dosimetry Techniques for Photodynamic Therapy such as LED sources including among others dye lasers, diode lasers and lamps (i.e. xenon arc lamps).

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### General consideration

The suppliers of the ingredients in the topical compositions are as follows:

### Example 1:

Phospholipids: Lipoid GmbH
Tris-Methylglucammonium Chlorin E6: Khimfarsintez LLC
Zincidone: UCIB - SOLABIA GROUP
Euxyl PE 9010: Schülke & Mayr GmBH
Glycerin - Meridional TCS Indústria e Comércio de Óleos S/A
Aristoflex AVC - Clariant International Limited
Green Grass (Parfum) - Düllberg Konzentra GmbH & Co. KG

### Example 2:

Refined Glycerin USP - Meridional TCS Indústria e Comércio de Óleos S/A
Crodamol (PPG-3 Benzyl Ether Myristate) - Croda
ViscOptima SE (Sodium Polyacrylate (and) Ethylhexyl Cocoate (and) PPG-3 Benzyl Ether
Myristate (and) Polysorbate 20) - Croda
Tocopherol Acetate - BASF
Vitis Vinifera (Grape) Seed Oil - Borges Agricultural & Industrial Edible Oils S.A.U.
Adansonia Digitata (Baobab) Seed Oil - B'Ayoba (Pvt) Ltd
Hydroxyapatite - NPF Medkom
Euxyl PE 9010 (Ethylhexylglycerin, Phenoxyethanol): Schülke & Mayr GmBH
Black Sea (Parfum) - CPL Aromas
Poloxamer 407 - Xi'An Imaherb Biotech Co. Ltd.
Zincidone: UCIB - Solabia Group
Tris-Methylglucammonium Chlorin E6: Khimfarsintez LLC

### Example 3:

Refined Glycerin USP - Meridional TCS Indústria e Comércio de Óleos S/A
ViscOptima SE (Sodium Polyacrylate (and) Ethylhexyl Cocoate (and) PPG-3 Benzyl Ether
Myristate (and) Polysorbate 20) - CRODA
Crodamol (PPG-3 Benzyl Ether Myristate) - Croda
Tocopherol Acetate - BASF
Vitis Vinifera (Grape) Seed Oil - Borges Agricultural & Industrial Edible Oils S.A.U.
Citrullus lanatus seed oil - - B'Ayoba (Pvt) Ltd
Zincidone: UCIB - Solabia Group
Tris-Methylglucammonium Chlorin E6: Khimfarsintez LLC
Green Grass (Parfum) - Düllberg Konzentra GmbH & Co. KG

### 1. Topical compositions of the present invention (Examples 1-3)

The compositions of the present invention (Examples 1-3) illustrate topical compositions of the present invention in form of gel, fluid, and cream, which comprise the combination of chlorine e6 trismeglumine salt and the zinc salt of *L*-pyroglutamine acid. The quantitative topical formulations of the compositions of Examples 1-3, where the amount of the ingredients is expressed in % by weight of the ingredient in relation to the total weight of the composition are disclosed herein below.

### Example 1: Topical Gel

| **Commercial name** | **INCI name** | **Function** | **% (w/w)** |
|---|---|---|---|
| Colloid of liposomes of chlorine e6 trismeglumine salt and | Aqua | Solvent | 90.4 |
| | Phospholipids | Active ingredient and | 3.00 |
| Zn PCA | | solvent | |
| | trismeglumine salt of Chlorin e6 | Active ingredient | 0.4⁽¹⁾ |
| | Zn PCA ⁽²⁾ | Active ingredient | 0.15 |
| Refined Glycerin USP | Glycerin | Emollient | 3.00 |
| Carbo mer | Ammonium Acryloyl dimethyltaurate/VP Copolymer | Rheology modifier | 2.00 |
| Euxyl PE 9010 | Ethylhexylglycerin, Phenoxyethanol | Preservative | 0.85 |
| Green Grass | Parfum | Parfum | 0.2 |

| | | | |
|---|---|---|---|
| (1) The percentage by weight refers to the amount of free chlorine e6 in the composition (2) The term Zn-PCA refers to zinc salt of L-pyroglutamine acid | | | |

The process for the preparation of the composition of Example 1 is disclosed herein below:
Step A: Preparation process of the colloid of liposome of chlorine e6 trismeglumine salt and Zn PCA
   1) dissolving 4 g of trismeglumine salt of chlorin e6 in 100 ml of water in a reactor.
   2) dispersing 30 g of phospholipids in the resulting solution of trismeglumine salt of chlorin e6 of step 1.
   3) Homogenizing the resulting colloid obtained in step 2 in a LEMP installation (linear magnetic transducer, serial number No. 1). Homogenization was carried out by chaotic movement of the mixed particles in the working cell (the height of the working area of the cell was 155 mm) and the flow rate was 30 l/h.
   4) In a separate container, dissolving the preservative in 700 ml of water.
   5) mixing the colloid obtained in step 3 and the preservative solution obtained in step 4.
   6) Adding water to the mixture obtained in step 5 until having a volume of 1 litter.
   7) The resulting liposomes obtained in step 6 were homogenized on a special LEMP unit (linear magnetic transducer, serial number No. 1). Homogenization was carried out by chaotic movement of the mixed particles in the working cell (the height of the working area of the cell was 155 mm) and the flow rate was 30 I/h.
   8) adding into the solution of liposomes obtained in step 7 in the working cell, Zn PCA and homogenize the colloid with a flow rate of 12 I/h.
Step B: preparation process of the gel composition
   1). Introducing in a reactor the liposomal solution of trismeglumine salt of chlorin e6 and Zn PCA obtained in step A.
   2). Adding the rheology modifier and mixing at a speed of 60 rpm until the rheology modifier was completely swollen.
   3). Adding the emollient and stirring at 60 rpm for 20 minutes.
   4). Introducing the fragrance and stirring at 60 rpm for 20 minutes.
   5). Measuring the pH level of the mixture obtained in previous step 4 using a pH meter and adjust the pH (if required with the addition of triethanolamine) until being from 6.9 to 7.4.

### Example 2: Topical Day fluid

| **Phase** | **Nº** | **Function** | **INCI name** | **% (w/w)** |
|---|---|---|---|---|
| Phase A | 1 | Solvent | Aqua | q.s. for 100⁽³⁾ |
| | 2 | Emollient | Glycerin | 3.00 |
| Phase B | 3 | Emollient | PPG-3 Benzyl Ether Myristate | 7.00 |
| | 4 | Emulsifying agent | Sodium Polyacrylate (and) Ethylhexyl Cocoate (and) PPG-3 Benzyl Ether Myristate (and) Polysorbate 20 | 2.00 |
| | 5 | Antioxidant | Tocopheryl Acetate | 0.05 |
| | 6 | Emollient | Vitis Vinifera (Grape) Seed Oil | 1.50 |
| | 7 | Emollient | Adansonia Digitata Seed Oil. | 0.65 |
| Phase C | 8 | stabilizers | Hydroxyapatite | 0.60 |
| | 9 | Preservative | Ethylhexylglycerin, Phenoxyethanol | 0.85 |
| | 10 | Parfum | Parfum | 0.20 |
| Phase D | 11 | Solvent | Aqua | 10.00 |
| | 12 | solubilizers and stabilizers | Poloxamer-407 | 2.00 |
| | 13 | Active ingredient | Zinc PCA⁽²⁾ | 0.15 |
| | 14 | Active ingredient | trismeglumine salt of Chlorin e6 | 0.05⁽¹⁾ |

| | | | | |
|---|---|---|---|---|
| (1) The percenatge by weight refers to the amount of free chlorine e6 in the composition (2) The term Zn-PCA refers to zinc salt of L-pyroglutamine acid (3) q.s. stands for sufficient amount for 100% | | | | |

The process for the preparation of the composition of Example 2 is disclosed herein below:
1. Phase A: Water (1) and glycerin (2) were introduced into the reactor and heated to 75-80°C with stirring.
2. Phase B: Components (3), (4), (5), (6) and (7) were mixed in a separate reactor and heated to 75-80°C.
3. Pouring Phase B into Phase A at 75°C, set to maximum stirring speed for 20 minutes. Then the stirring speed was reduced to 100 rpm and the heating was turned off to obtain an oil-in-water emulsion.
4. In another separate container, mixing the Phase D components (11), (12), (13) and (14) until completely dissolution.
5. When the emulsion obtained in step 3 reached a temperature below 35°C, the mixture of components of phase D obtained in step 4 were added, and the resulting mixture was mixed at 100 rpm for 20 minutes.
6. Then, the components (8), (9) and (10) were added in succession and mixed at a speed of 100 rpm for 20 minutes.
7. Measuring the pH level of the mixture obtained in previous step 6 using a pH meter and adjust the pH (if required with the addition of triethanolamine) until being from 6.6-7.0.

### Example 3: Topical Day cream

| **Nº** | **Phase** | **Function** | **INCl name** | **% (w/w)** |
|---|---|---|---|---|
| 1 | Phase A | Solvent | Aqua | 48.65 |
| 2 | | Emollient | Glycerine | 5 |
| 3 | Phase B | Emulsifying agent | Butyl Alcohol, Stearic Acid, Lecithin, Caprylic/Capric Triglyceride | 3.5 |
| 4 | | Emulsifying agent | Glycerol monostearate | 1.2 |
| 5 | | Emollient | Isopropylisostearate | 3.1 |
| 6 | | Emollient | Capric/caprylic triglycerides | 3 |
| 7 | | Emollient | Vitis vinifera(Grape) seed oil | 2.9 |
| 8 | | Emollient | Citrullus lanatus Seed Oil | 1.5 |
| 9 | | Emollient | Adansonia Digitata Seed Oil | 1.5 |
| 10 | | Emollient | Stearyl Heptanoate | 5 |
| 11 | Phase C | Emulsifying agent | Ammonium Acryloyldimethyltaurate / VP Copolymer | 0.8 |
| 12 | Phase D | Solvent | Aqua | 20 |
| 13 | | solubilizers and stabilizers | Poloxamer-407 | 2 |
| 14 | | Active ingredient | Zn PCA⁽²⁾ | 0.2 |
| 15 | | Active ingredient | trismeglumine salt of Chlorine e6 | 0.05⁽¹⁾ |
| 16 | Phase E | Preservative | Phenoxyethanol (and) Ethylhexylglycerin | 0.85 |
| 17 | | Parfum | Parfum | 0.15 |
| 18 | | Stabilizers | Hydroxyapatite | 0.6 |

| | | | | |
|---|---|---|---|---|
| (1) The percentage by weight refers to the amount of free chlorine e6 in the composition (2) The term Zn-PCA refers to zinc salt of L-pyroglutamine acid | | | | |

The process for the preparation of the composition of Example 3 is disclosed herein below:
1. Phase A. Water (1) and glycerin (2) were introduced into the reactor and heated to 75-80°C with stirring.
2. Phase B. Components (3), (4), (5), (6), (7), (8), (9) and (10) were mixed in a separate reactor and heated to 75-80°C.
3. Pouring Phase B into Phase A at 75°C, set to maximum stirring speed for 20 minutes. Then the stirring speed was reduced to 100 rpm and the heating was turned off to obtain an oil-in-water emulsion.
4. At a temperature of 50-45°C, component (11) is introduced into the emulsion obtained in step 3 with constant stirring at a speed of 100 rpm. Stirring was carried out until complete dissolution and swelling of the mass.
5. In another separate container, mixing the Phase D components (12), (13), (14) and (15) until completely dissolved.
6. When the emulsion obtained in step 4 reached a temperature below 35°C, the mixture of components of phase D obtained in step 5 were added, and the resulting mixture was mixed at 100 rpm for 20 minutes.
7. Then, the components (16), (17) and (18) were introduced sequentially and mixed for 20 minutes at a speed of 100 rpm.
8. Measuring the pH level of the mixture obtained in previous step 6 using a pH meter and adjust the pH (if required with the addition of triethanolamine) until being from pH = 6.8 - 7.3.

### 2. In vivo assay

### 2.1. Patients

Patients suffering from severe inflammatory acne received a facial phototherapy comprising the composition of the present invention. In particular, the patients included in the assay were females from 18 to up to 30 years old, with acne-skin condition, presenting open and closed comedones, as well as inflammatory elements on a hyperemic background. Acne scars are also present.

Prior to the sessions, it was verified that the patients did not present any known exclusion criteria from performing phototherapy.

### Exclusion criteria

Patients suffering from epilepsy, hereditary or acquired porphyria, increased skin photosensitivity caused by photosensitizing medication, cardiovascular and respiratory failure, liver, and kidney diseases in the stage of decompensation, systemic lupus erythematosus, cachexia, fever, pregnancy and lactation, acute inflammatory processes, blood diseases, mental illness, and/or idiosyncrasy.

### 2.2. Phototherapy treatment

The phototherapy involved 5 facial sessions within one month. Each patient was treated on half of the face with the phototherapy treatment comprising the composition of the present invention and the other half of the face with a comparative phototherapy treatment without the composition of the present invention.

The phototherapy schedule of the present invention and the comparative phototherapy schedule falling outside the scope of the present invention are disclosed herein below.

### 2.2.1. Phototherapy treatment with the composition of the present invention:

### 1. Pre-treatment

Prior to the light irradiation the following cleansing treatment was performed:
Firstly, a mild facial cleanser was applied; and
Secondly, a mild enzymatic exfoliant gel was applied and left on the skin to remove any dead skin cell.

### 2. Photosensitizing step

The gel composition of the present invention Example 1 was applied to one half of the face.

### 3. Phototherapy

The phototherapy involved irradiating the skin in **each session** with blue LED light at 420nm ±10nm (Blue or B LED light), red light at 630-660nm ±10nm, and in part of the sessions also yellow light 590 nm ±10nm as follows. The protocol of each phototherapy session is disclosed herein below wherein B is blue LED light; R is red LED light and Y refers to yellow LED light being followed by a number expression the time of exposure in minutes:

| **Session number** | **Patient 1** | **Patient 2** |
|---|---|---|
| 1 | B15 + R15 + Y5 | B10 + R20 |
| 2 | B10 + R15 | B15 + R15 |
| 3 | B10 + R20 | B15 + R15 |
| 4 | B10 + R20 + Y5 | B15 + R15 |
| 5 | B10 + R20 + Y5 | B15 + R15 |

### 2.2.2. Comparative phototherapy treatment without the composition of the present invention

### 1. Pre-treatment

Prior to the light irradiation the following cleansing treatment was performed:
Firstly, a mild facial cleanser was applied; and
Secondly, a mild enzymatic exfoliant gel was applied and left on the skin to remove any dead skin cells.

### 2. Phototherapy

The phototherapy involved blue LED light at 420nm ±10nm and red light at 630-660nm ±10nm.

### 2.3. Results

After the 5 sessions of phototherapy, an improved general regression of the treated side of the face was observed in comparison with the untreated side of the face. In particular, a significant reduction of the inflammatory eruptions, and stronger improvement of the resorption of closed comedones was observed. Further, a significant improvement in the microrelief, skin texture and increased luminosity in the half of the face treated with the composition of the present invention was also observed in comparison to the untreated side of the skin face.

There was also a considerable improvement in complexion and smoothing of scar tissue in the treated side of the face. In fact, the rash on the treated side of the face was completely stopped. Meanwhile, the untreated side of the face still has rash and a nonsignificant ameliorant of the skin texture was observed. Finally, according to the patient's evaluation, the skin had become more elastic and a generalized improvement of the skin was observed after a few sessions increasing the adhesion of the treatment and the reduction of abandonments of the therapy.

Therefore, considering the above results, it can be concluded that the composition of the present invention has an enhanced unexpected effect on skin, and particularly as skin care agent and also in those skins suffering from a disease or disorder that involves Inflammation, excessive sebum production, inflammation, and/or microbiome imbalances.

### Citation List

1. US2016213781
2. Article 2, Regulation (EU) 2017/745 of The European Parliament and of The Council of 5. April 2017.
3. Annex XVI, Regulation (EU) 2017/745 of The European Parliament and of The Council of 5 April 2017.

## Claims

1. A topical composition comprising:
a) an effective amount of 18-carboxy-20- (carboxymethyl) -8-ethenyl-13-ethyl-2,3-dihydro-3,7,12,17-tetramethyl 21H, 23H porphin-2-propionic acid of formula (I): 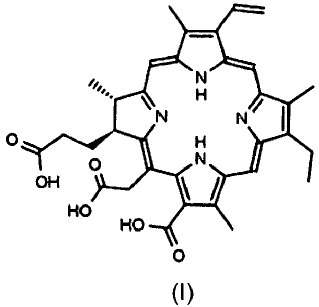 or a topically acceptable salt thereof; or a topically acceptable liposome containing it;
b) an effective amount of zinc salt of *L*-pyroglutamine acid of formula (V) 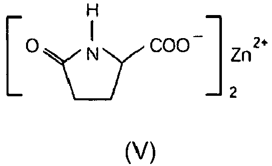 ; and
c) optionally, one or more topically acceptable excipients or carriers.

2. The topical composition according to claim 1, wherein the 18-carboxy-20 (carboxymethyl) -8-ethenyl-13-ethyl-2,3-dihydro-3,7,12,17-tetramethyl 21H, 23H porphin-2-propionic acid (a) is in form of a salt selected from the group consisting of an alkaline metal salt, an alkaline earth metal salt, trimeglumine salt, or mixture thereof;
particularly, a salt selected from the group consisting of:
the trisodium salt of formula (II) 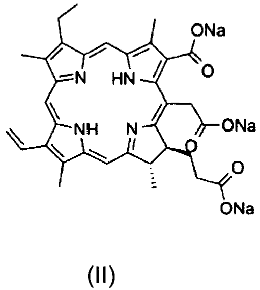
and the trimeglumine salt of formula (III)

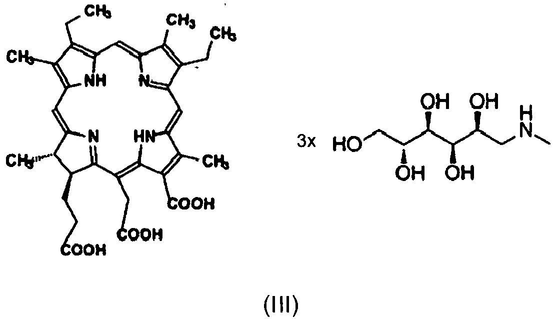

3. The topical composition according to claim 1, wherein the 18-carboxy-20 (carboxymethyl) -8-ethenyl-13-ethyl-2,3-dihydro-3,7,12,17-tetramethyl 21H, 23H porphin-2-propionic acid (a) is in form of a topically acceptable liposome containing it.

4. The topical composition according to any of the claims 1-3, comprising:
a) From 0.01% to 1% by weight of 18-carboxy-20 (carboxymethyl) -8-ethenyl-13-ethyl-2,3-dihydro-3,7,12,17-tetramethyl 21H, 23H porphin-2-propionic acid, or a topically acceptable salt thereof, or a topically acceptable liposome containing it; and
b) From 0.01% to 2 % by weight of the zinc salt of *L*-pyroglutamine acid.

5. The composition according to any of the claims 1-4, comprising:
a) From 0.03% to 0.6% by weight of 18-carboxy-20 (carboxymethyl) -8-ethenyl-13-ethyl-2,3-dihydro-3,7,12,17-tetramethyl 21H, 23H porphin-2-propionic acid, or a topically acceptable salt thereof; or a topically acceptable liposome containing it; and
b) From 0.05% to 0.3% by weight of the zinc salt of *L*-pyroglutamine acid.

6. The composition according to any of the claims 1-5, comprising one or more topically acceptable excipients or carrier selected from the group consisting of solvent, co-solvent, emollient, humectant, emulsifying agent, stabilizer, preservative, parfum, antioxidant, rheology modifier agent, pH regulator agent and gelling agent.

7. The composition according to any of the claims 1-6, which is in a form selected from the group consisting of emulsion, cream, gel, fluid, and lotion.

8. The composition according to any of the claims 1-7, which is a pharmaceutical composition comprising:
a) a therapeutically effective amount of chlorine e6, or a topically acceptable salt thereof; or a topically acceptable liposome containing it;
b) a therapeutically effective amount of zinc salt of *L*-pyroglutamine acid; and
c) optionally, one or more topically pharmaceutically acceptable excipients or carriers; for use in therapy.

9. The composition for use according to claim 8, in the treatment of a skin disease or disorder involving one or more of the following symptoms: inflammation, excessive sebum production, hyperpigmentation, and microbiome imbalances.

10. The composition for use according to claim 9, in the treatment of a skin disease or disorder selected from the group consisting of acne, rosacea, eczema, psoriasis, and hyperpigmentation.

11. Use of the composition as defined in any of the claims 1-7, which is a cosmetic composition comprising:
a) a cosmetically effective amount of chlorine e6, or a topically acceptable salt thereof; or a topically acceptable liposome containing it;
b) a cosmetically effective amount of zinc salt of *L*-pyroglutamine acid; and
c) optionally, one or more topically cosmetically acceptable excipients or carriers; in cosmetic.

12. The use of the composition according to claim 10, as a skincare agent; particularly wherein the skincare comprises ameliorating at least one of the following symptoms: roughness, flakiness, dehydration, tightness, chapping, lack of elasticity, and dull skin.

13. The composition for use according to any of the claims 8-10, where the composition is a photosensitizing agent; particularly in a therapeutic photodynamic therapy; or alternatively,
the use according to any of the claims 11-12, wherein the composition is a photosensitizing agent; particularly in a cosmetic photodynamic therapy.

14. The composition for use according to claim 13, wherein the photodynamic therapy comprises exposing the skin area to be treated to light having a wavelength from 400 to 760 nm.

15. A kit comprising:
the composition as defined in any of the claims 1-7;
optionally, means to apply the composition to the area to be treated; and
optionally, a light emission source.
